# EUROPÄISCHE PATENTANMELDUNG

(11) **EP 0 772 044 A2**
(43) Veröffentlichungstag der Anmeldung: **07.05.1997**
(21) Anmeldenummer: 96113971.4
(22) Anmeldetag: 31.08.1996
(51) Int. Cl.: G01N 33/558, G01N 33/543, G01N 33/72, G01N 33/76

(54) **Testvorrichtung und Testverfahren**

(30) Priorität: 31.10.1995 DE 19540541
(71) Anmelder: Human Gesellschaft für Biochemica und Diagnostica mbH, 65232 Taunusstein-Wehen (DE)
(72) Erfinder: Dreher, Michael Dr., 56357 Welterod (DE); Schell, Dietmar Dr., 65185 Wiesbaden (DE)
(74) Vertreter: Weber, Dieter, Dr.

(57) **Zusammenfassung**

Eine Testvorrichtung mit einem porösen saugfähigen Träger, der in einem ersten Bereich (1a) mit einem an einen Analyten bindenden ersten spezifischen Bindungsreagenz, das in feuchtem Zustand des Trägers in diesem frei beweglich ist, imprägniert ist und in einem zweiten Bereich (2a) ein auf dem Träger immobilisiertes, an denselben Analyten bindendes zweites spezifisches Bindungsreagenz aufweist, ist dadurch gekennzeichnet, daß der Träger in einem von dem ersten Bereich (1a) räumlich getrennten, zwischen dem ersten und dem zweiten Bereich (2a) liegenden dritten Bereich (1b) mit einem mit dem ersten spezifischen Bindungsreagenz reagierenden signalgebenden Stoff imprägniert ist, der in feuchtem Zustand des Trägers in diesem frei beweglich ist. Bei der Verwendung bringt man die Testvorrichtung mit einer wäßrigen Flüssigkeitsprobe, die möglicherweise den Analyten enthält, derart in Kontakt, daß die Flüssigkeitsprobe von dem ersten über den dritten durch den zweiten Bereich des Trägers hindurchgesaugt wird, und prüft, ob und gegebenenfalls in welchem Ausmaß in dem zweiten Bereich des Trägers ein Signal auftritt.

## Beschreibung

Die Verwendung von mit Reagenzien imprägnierten Teststreifen zum Nachweis von Analyten, wie beispielsweise Schwangerschaftshormonen, in wäßrigen Flüssigkeitsproben ist seit längerer Zeit bekannt. Bei diesen bekannten Verfahren wird üblicherweise die Flüssigkeitsprobe mit einem Teststreifen in Kontakt gebracht, der aus einem porösen saugfähigen Träger besteht oder einen solchen enthält, der in einem ersten Bereich mit einem an den Analyten bindenden ersten spezifischen Bindungsreagenz, das in feuchtem Zustand des Trägers in diesem frei beweglich ist, imprägniert ist und in einem zweiten Bereich ein auf dem Träger immobilisiertes, an den gleichen Analyten bindendes zweites spezifisches Bindungsreagenz aufweist. Das erste immobile Bindungsreagenz ist bei diesem Verfahren seinerseits an eine signalgebende Komponente gebunden. Bringt man nun den Teststreifen mit der Flüssigkeitsprobe in Kontakt, so wird die Flüssigkeitsprobe in den ersten Bereich des Trägers eingesaugt, wo der Analyt mit dem ersten spezifischen Bindungsreagenz, an welches die signalgebende Komponente gebunden ist, reagiert.

Der dabei gebildete Komplex wandert mit Hilfe der in dem Träger weitergesaugten Flüssigkeitsprobe zu dem immobilisierten zweiten Bindungsreagenz, mit welchem der Analyt reagiert, so daß in dem Zweiten Bereich des Trägers, der als Nachweiszone dient, die dort immobilisierte signalgebende Komponente festgestellt werden kann.

Bei den spezifischen Bindungsreagenzien kann es sich zum Beispiel um spezifische Antikörper, Antigene oder auch andere Reagenzien handeln, die in der Lage sind, mit dem nachzuweisenden Analyten eine Bindung einzugehen. Bei den signalgebenden Komponenten kann es sich um Radioisotope, vorzugsweise aber um Farbstoffe, gefärbte Partikel oder Partikel mit Eigenfärbung handeln. Je nach der verwendeten signalgebenden Komponente ist in der Nachweiszone der Analyt visuell oder durch geeignete Geräte zu bestimmen. Das Signal kann entweder rein qualitativ oder auch semiquantitativ oder quantitativ ausgewertet werden. Beispiele für Druckschriften, die solche Verfahren beschreiben, sind die GB-PS 1 589 234, die EP-PS 0 225 054, die EP-PS 0 183 442, die EP-PS 1 086 799, die EP-PS 0 291 194, die WO 88/08 534 sowie der Aufsatz von Horton et al in J. Immun. Meth. 140 (1991), Seiten 131 bis 134.

Das Ziel solcher Testverfahren ist, einen Analyten in möglichst kurzer Zeit ohne aufwendige Methodik mit möglichst hoher Sensitivität nachzuweisen. Der schwerwiegende Nachteil der bekannten Verfahren für visuellen Nachweis bestimmter Analyten beteht jedoch darin, daß als signalgebende Komponente entweder relativ große gefärbte Partikel (z. B. Polystyrol, Polymethacrylsäuremethylester, Kombinationspartikel mit einem Kern und einer Schale oder Aktivkohle) oder aber sehr kleine Partikel (z. B. Metallsole) eingesetzt werden. Während die Verwendung großer Partikel den Vorteil besserer visueller Erkennung bietet, ist bei ihnen die Reaktionsgeschwindigkeit und Beladung unerwünscht niedrig, während bei Verwendung kleiner Partikel die Reaktionsgeschwindigkeit und Beladung höher, die visuelle Erkennung allerdings vermindert ist. Hierzu wird auf Bangs, The Latex Course (1993), Seite 21 hingewiesen. Mit den oben beschriebenen bekannten Verfahren werden daher heute Nachweisgrenzen von nur etwa 0,1 bis 0,2 nmol/l erreicht (wie bei typischen handelsüblichen Schwangerschaftstests zum Nachweis des Hormons hCG). Ein Hersteller solcher Testvorrichtungen zum Nachweis dieses Hormons gibt an, daß 25 IU/l nur mit fünfzigprozentiger Sicherheit erkannt werden können, während für eine eindeutige Erkennbarkeit 50 IU/l erforderlich sind.

Die der Erfindung zugrundeliegende Aufgabe bestand daher darin, diese Nachteile des Standes der Technik auszuräumen und eine Testvorrichtung und ein Testverfahren zu bekommen, die ohne aufwendige Methodik rasch eine visuelle Feststellung des nachzuweisenden Analyten mit hoher Sensitivität erlauben.

Diese Aufgabe wird mit der erfindungsgemäßen Testvorrichtung und dem erfindungsgemäßen Testverfahren gelöst.

Die erfindungsgemäße Testvorrichtung mit einem porösen saugfähigen Träger, der in einem ersten Bereich mit einem an einen Analyten bindenden ersten spezifischen Bindungsreagenz, das in feuchtem Zustand des Trägers in diesem frei beweglich ist, imprägniert ist und in einem zweiten Bereich ein auf dem Träger immobilisiertes, an den gleichen Analyten bindendes zweites spezifisches Bindungsreagenz aufweist, ist dadurch gekennzeichnet, daß der Träger in einem von dem ersten Bereich räumlich getrennten, zwischen dem ersten und dem zweiten Bereich liegenden dritten Bereich mit einem mit dem ersten spezifischen Bindungsreagenz reagierenden signalgebenden Stoff imprägniert ist, der in feuchtem Zustand des Trägers in diesem frei beweglich ist.

Das erfindungsgemäße Testverfahren zur Bestimmung der Anwesenheit eines Analyten ist dadurch gekennzeichnet, daß man eine Testvorrichtung mit den obigen Merkmalen mit einer wäßrigen Flüssigkeitsprobe, die möglicherweise den Analyten enthält, derart in Kontakt bringt, daß die Flüssigkeitsprobe von dem ersten über den dritten durch den zweiten Bereich des Trägers hindurch gesaugt wird, und prüft, ob und gegebenenfalls in welchem Ausmaß in dem zweiten Bereich des Trägers ein Signal auftritt.

Zweckmäßig ist es, in einem vierten Bereich des Trägers, der sich an den zweiten Bereich, die Nachweiszone, anschließt, ein drittes Bindungsreagenz vorzusehen, das zweckmäßig auf dem Träger immobilisiert ist und überschüssiges bzw. nicht mit dem Analyten zur Reaktion gebrachtes erstes spezifisches Bindungsreagenz und unumgesetzten signalgebenden Stoff festhält. Stattdessen oder zusätzlich kann im Anschluß an den zweiten Bereich des Trägers ein saugfähiger Bereich vorgesehen sein, der nichtfestgehaltenes Bindungsreagenz, signalgebende Komponente und Probenbestandteile aufnimmt.

Der Träger kann ein Streifen eines einheitlichen porösen saugfähigen Materials sein, sofern gewährleistet ist, daß das erste Bindungsreagenz und der signalgebende Stoff nicht an ihn gebunden sind oder mit ihm reagieren, während das zweite spezifische Bindungsreagenz an ihn bindbar und damit immobilisierbar ist. Da in bestimmten Fällen die Gefahr besteht, daß beispielsweise der signalgebende Stoff mit dem porösen Träger reagiert, wenn dieser geeignet ist, andererseits das zweite spezifische Bindungsreagenz zu immobilisieren, kann es zweckmäßig sein, den Träger aus mehreren unterschiedlichen Abschnitten zu bilden, wobei der erste Abschnitt für das erste spezifische Bindungsreagenz und den signalgebenden Stoff ein poröser Filtermaterialstreifen, wie Filterpapier, ist, während ein zweiter, sich daran anschließender Abschnitt des Trägers, an den das zweite und gegebenenfalls dritte Bindungsreagenz gebunden sind, um sie darauf zu immobilisieren, aus einer Membran besteht. Zweckmäßig schließt sich an diese Membran wiederum ein Filtermaterialabschnitt an, der die Flüssigkeitsprobe durch den Bereich des Membranabschnittes hindurchsaugt und nichtfestgehaltene Reagenzien und Probenbestandteile aufnimmt.

Die Filterabschnitte bestehen zweckmäßig aus Cellulose, einem Cellulosederivat oder einem Mischgewebe aus Cellulose und polymeren Amiden oder Estern. Die Membran besteht bevorzugt aus nitrierter Cellulose. Letztere hat zweckmäßig eine Porengröße von 3 bis 20 µm, besonders bevorzugt im Bereich von 8 bis 12 µm. Die verschiedenen Abschnitte des Trägers können sich auf einem gemeinsamen Stützträger abstützen und darauf befestigt sein.

In der erfindungsgemäßen Testvorrichtung und dem mit ihr durchgeführten Testverfahren sind die Analyten zweckmäßig Antigene, Haptene, Antikörper oder andere diagnostisch relevante Stoffe aus Körperflüssigkeiten. Beispiele der nachweisbaren Analyten sind das Schwangerschaftshormon Choriongonadotropin (hCG), menschliche Antigene oder menschliches Hämoglobin. Der Bindungsmechanismus für den Analyten bei der erfindungsgemäßen Testvorrichtung erfolgt nach dem bekannten "Sandwich"-Mechanismus.

Als erstes spezifisches Bindungsreagenz werden zweckmäßig Antikörper, Antigene, Haptene, Rezeptoren, Peptide, Lectine, Glycoproteine und Oligo- oder Polysaccharide benutzt. Diese Stoffe kommen auch als zweites und drittes Bindungsreagenz in Betracht. Bei Verwendung von Antikörpern können diese polyklonale oder monoklonale Antikörper sein. Das erste spezifische Bindungsreagenz besitzt wenigstens eine Bindungsstelle zur Bindung an die signalgebende Komponente, wobei diese Bindungsstelle vorzugsweise aus Biotin oder Fluorescein besteht.

Die signalgebende Komponente kann bekanntermaßen ein Radioisotop sein, was jedoch zur Folge hat, daß der Nachweis des Analyten nicht direkt mit dem bloßen Auge erfolgen kann, sondern ein Gerät erfordert. Zweckmäßig ist daher der signalgebende Stoff ein Farbstoff, ein gefärbtes partikuläres Reagenz oder ein Metallsol, die jeweils mindestens eine Bindungsstelle zur Bindung an das erste spezifische Bindungsreagenz aufweisen. Zweckmäßig bestehen die Bindungsstellen des signalgebenden Stoffes aus Avidin, Streptavidin bzw.Antikörper gegen Biotin oder Derivaten mit vergleichbarer Bindungsaffinität zu Biotin bzw. anti-Fluorescein mit vergleichbarer Bindungsaffinität zu Fluorescein. Der signalgebende Stoff ist zweckmäßig gefärbter Polystyrollatex mit einem Teilchengrößenbereich von 0,05 bis 3 µm, zweckmäßig mit einem Teilchengrößenbereich von 0,2 bis 0,5 µm.

Die Zeichnung zeigt einen senkrechten Schnitt durch eine bevorzugte Ausführungsform einer Testvorrichtung nach der Erfindung. Diese Testvorrichtung besteht aus einem Träger für die Bindungsreagenzien und dem signalgebenden Stoff. Dieser Träger besitzt ein Stützteil 4 in Form eines länglichen Streifens. Auf diesem Stützteil 4 sind ein Filtermaterialstreifen 1, eine ihn überlappende Membran 2 und ein daran anschließender Filterstreifen 3 befestigt. In der Position 1a ist das Filtermaterial mit dem ersten spezifischen Bindungsreagenz und in der Position 1b mit dem signalgebenden Stoff imprägniert. In der Position 2a ist an die Membran 2 das zweite spezifische Bindungsreagenz und in der Position 2b das dritte Bindungsreagenz unter Immobilisierung gebunden. Der Filtermaterialstreifen 1 besteht aus vorbehandelter Cellulose, die Membranschicht 2 aus nitrierter Cellulose und der Filtermaterialstreifen 3 aus unvorbehandelter Cellullose.

### Beispiel 1

### Herstellung eines Teststreifens für den Nachweis von humanem Choriongonadotropin (hCG)

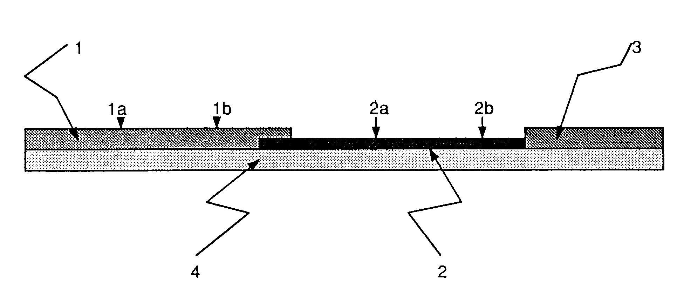

Ein Teststreifen nach Abb. 1, bestehend aus einem Trägermaterial 4, das eine Membranschicht aus nitrierter Cellulose 2 sowie einen mit unspezifischen Proteinen, wie Schaf-IgG und Rinderalbumin, vorbehandelten Cellulosefilter 1 und einen unvorbehandelten Cellulosefilter 3 enthält, wird hergestellt.

Auf das vorbehandelte Cellulosefilter 1 werden im unteren Teil spezifische Antikörper gegen hCG, die eine Biotin-Markierung tragen, aufgebracht (1a). Im oberen Teil ist eine signalgebende Komponente, hier blau gefärbter Polystyrollatex, aufgebracht, die mit Streptavidin beschichtet ist (1b). Die Membranschicht 2 trägt fixierte Bindungsreagenzien, die den Analyten 2a und die signalgebende Komponente nach Reaktion mit den biotinylierten anti-hCG Antikörpern binden (2b). Während der Reaktion wird die Probe mit dem nachzuweisenden hCG (A) zunächst von dem vorbehandelten Filter 1 aufgenommen und in Richtung der Membranschicht 2 transportiert.

Während dieses Transportes reagiert der Analyt hCG (A) aus der Probe mit den spezifischen Antikörpern 1a. Der dadurch gebildete lösliche Komplex sowie nichtreagierte Antikörper 1a binden anschließend an die Streptavidin-Gruppen des Polystyrollatex. Die Verbindung aus Analyt, Antikörper und Polystyrollatex wandert weiter über die Membran 2 und wird hier zunächst von den spezifischen anti-hCG Antikörpern 2a gebunden. Überschüssige Verbindung und solche ohne gebundenes hCG werden danach von einem dritten Antikörper 2b, spezifisch für den biotinylierten anti-hCG Antikörper, gebunden. Die Anwesenheit von hCG in der Probe kann visuell mit dem bloßen Auge bis zu 2 IU/l (entspricht ca. 0,0067 nmol/l) nachgewiesen werden.

| **Ergebnis** | |
|---|---|
| **hCG Konzentration, IU/l** | **Relative Intensität** |
| 0 | --- |
| 1 | --- |
| 2 | ((+)) |
| 3 | ((+)) |
| 4 | ((+)) |
| 5 | (+) |
| 10 | + |
| 20 | + |
| 30 | ++ |
| 50 | ++ |
| 100 | +++ |
| 10 000 | ++++ |
| ((+)) Signal sehr schwach, jedoch deutlich vom Hintergrund unterscheidbar (+) Signal schwach, aber klar erkennbar + Signal deutlich ++ Signal sehr deutlich +++ Signal stark ++++ Signal sehr stark | |

### Beispiel 2

### Herstellung eines Teststreifens für den Nachweis von humanem Hämoglobin

Ein Teststreifen nach der Abbildung wird wie in Beispiel 1 beschrieben hergestellt. Auf das vorbehandelte Cellulosefilter 1 werden im unteren Teil spezifische Antikörper gegen Hämoglobin, die eine Biotin-Markierung tragen, aufgebracht (1a). Im oberen Teil ist blau gefärbter Polystyrollatex aufgebracht, der mit Streptavidin beschichtet ist (1b). Die Membranschiciht 2 trägt fixierte Antikörper gegen Hämoglobin 2a, sowie Antikörper gegen die biotinylierten anti-Hämoglobin-Antikörper 2b.

Während der Reaktion wir die Probe mit dem nachzuweisenden Hämoglobin zunächst von dem vorbehandelten Filter 1 aufgenommen und in Richtung der Mambranschicht 2 transportiert. Während dieses Transports reagiert Hämoglobin (A) aus der Probe mit den biotinylierten anti-Hämoglobin-Antikörpern 1a. Der dadurch gebildete lösliche Komplex sowie nichtreagierte Antikörper 1a binden anschließend an die Streptavidin-Gruppen des Polystyrollatex. Die Verbindung aus Hämoglobin, Antikörper und Polystyrollatex wandert weiter über die Membran 2 und wird hier zunächst von den spezifischen anti-Hämoglobin-Antikörpern 2a gebunden. Überschüssige Verbindung und solche ohne gebundenes Hämoglobin werden danach von einem weiteren Antikörper 2b spezifisch für den biotinylierten anti-Hämoglobin-Antikörper gebunden.

Die Anwesenheit von Hämoglobin in der Probe kann visuell mit dem bloßen Auge bis zu 0,01 µg/ml (entspricht ca. 0,147 nmol/l) nachgewiesen werden.

| **Ergebnis** | |
|---|---|
| **Hämoglobin-Konzentration, µg/l** | **Relative Intensität** |
| 0 | --- |
| 0,01 | ((+)) |
| 0,05 | (+) |
| 0,1 | (+) |
| 0,5 | ++ |
| 1 | ++ |
| 10 | ++++ |
| 100 | +++ |
| 1000 | (+) |
| ((+)) Signal sehr schwach, jedoch deutlich vom Hintergrund unterscheidbar (+) Signal schwach, aber klar erkennbar + Signal deutlich ++ Signal sehr deutlich +++ Signal stark ++++ Signal sehr stark | |

## Patentansprüche

1. Testvorrichtung mit einem porösen saugfähigen Träger, der in einem ersten Bereich (1a) mit einem an einen Analyten bindenden ersten spezifischen Bindungsreagenz, das in feuchtem Zustand des Trägers in diesem frei beweglich ist, imprägniert ist und in einem zweiten Bereich (2a) ein auf dem Träger immobilisiertes, an denselben Analyten bindendes zweites spezifisches Bindungsreagenz aufweist, **dadurch gekennzeichnet**, daß der Träger in einem von dem ersten Bereich (1a) räumlich getrennten, zwischen dem ersten und dem zweiten Bereich (2a) liegenden dritten Bereich (1b) mit einem mit dem ersten spezifischen Bindungsreagenz reagierenden signalgebenden Stoff imprägniert ist, der im feuchten Zustand des Trägers in diesem frei beweglich ist.

2. Testvorrichtung nach Anspruch 1, **dadurch gekennzeichnet**, daß ein sich an den zweiten Bereich (2a) des Trägers anschließender vierter Bereich (2b) des Tägers ein drittes Bindungsreagenz enthält, das an nicht mit dem Analyten umgesetztes Bindungsreagenz und signalgebende Komponente bindet.

3. Vorrichtung nach Anspruch 2, **dadurch gekennzeichnet**, daß das dritte Bindungsreagenz auf dem Träger immobilisiert ist.

4. Testvorrichtung nach einem der Ansprüche 1 bis 3, **dadurch gekennzeichnet**, daß sich an den zweiten Bereich (2a) bzw. vierten Bereich (2b) ein weiterer Bereich aus porösem saugfähigem Material anschließt.

5. Testvorrichtung nach einem der Ansprüche 1 bis 4, **dadurch gekennzeichnet**, daß der saugfähige Träger einen ersten Abschnitt aus Filtermaterial und einen sich daran anschließenden Membranabschnitt aufweist.

6. Testvorrichtung nach Anspruch 5, **dadurch gekennzeichnet**, daß sie anschließend an den Membranabschnitt einen weiteren Filtermaterialabschnitt aufweist.

7. Testvorrichtung nach einem der Ansprüche 5 oder 6, **dadurch gekennzeichnet**, daß die Filtermaterialabschnitte und der Membranabschnitt auf einem Stützmaterial befestigt sind.

8. Testvorrichtung nach einem der Ansprüche 5 bis 7, **dadurch gekennzeichnet**, daß das Filtermaterial des ersten Abschnittes mit unspezifischen Proteinen vorbehandelt und jenes des letzten Abschnittes unvorbehandelt ist.

9. Testvorrichtung nach einem der Ansprüche 1 bis 8, **dadurch gekennzeichnet**, daß das Filtermaterial aus Cellulose, einem Cellulosederivat oder einem Mischgewebe aus Cellulose und polymeren Amiden oder Estern besteht.

10. Testvorrichtung nach einem der Ansprüche 5 bis 9, **dadurch gekennzeichnet**, daß der Membranabschnitt aus nitrierter Cellulose besteht.

11. Testvorrichtung nach Anspruch 10, **dadurch gekennzeichnet**, daß die nitrierte Cellulose eine Porengröße von 3 bis 20, vorzugsweise 8 bis 12 µm besitzt.

12. Testvorrichtung nach einem der Ansprüche 1 bis 11, **dadurch gekennzeichnet**, daß die spezifischen Bindungsreagenzien an Antigene, Haptene, Antikörper oder andere diagnostisch relevante Stoffe aus Körperflüssigkeiten binden.

13. Testvorrichtung nach einem der Ansprüche 1 bis 12, **dadurch gekennzeichnet**, daß das erste und/oder zweite und/oder dritte Bindungsreagenz aus einem Antikörper, Antigen, Hapten, Rezeptor, Peptid, Lectin, Glycoprotein, Oligo- oder Polysaccharid besteht.

14. Testvorrichtung nach Anspruch 13, **dadurch gekennzeichnet**, daß wengistens eines der spezifischen Bindungsreagenzien ein polyklonaler oder monoklonaler Antikörper ist.

15. Testvorrichtung nach einem der Ansprüche 1 bis 14, **dadurch gekennzeichnet**, daß das erste spezifische Bindungsreagenz mindestens eine Bindungsstelle zur Bindung an den signalgebenden Stoff enthält.

16. Testvorrichtung nach Anspruch 15, **dadurch gekennzeichnet**, daß das erste spezifische Bindungsreagenz als Bindungsstelle Biotin oder Fluorescein besitzt.

17. Testvorrichtung nach einem der Ansprüche 1 bis 16, **dadurch gekennzeichnet**, daß sie als signalgebenden Stoff einen Farbstoff, ein gefärbtes partikuläres Reagenz oder Metallsol enthält, das jeweils mindestens eine Bindungsstelle zur Bindung an das erste spezifische Bindungsreagenz besitzt.

18. Testvorrichtung nach Anspruch 17, **dadurch gekennzeichnet**, daß sie als signalgebenden Stoff einen gefärbten Polystyrollatex mit einer Teilchengröße von 0,05 bis 3, vorzugsweise 0,2 bis 0,5 µm enthält.

19. Testvorrichtung nach Anspruch 17 oder 18, **dadurch gekennzeichnet**, daß sie einen signalgebenden Stoff enthält, dessen Bindungsstelle aus Avidin, Streptavidin, Antikörper gegen Biotin oder Derivaten mit vergleichbarer Bindungsaffinität zu Biotin bzw. anti-Fluorescein mit vergleichbarer Bindungsaffinität zu Fluorescein besteht.

20. Testverfahren zur Bestimmung der Anwesenheit eines Analyten, **dadurch gekennzeichnet**, daß man eine Testvorrichtung nach einem der Ansprüche 1 bis 19 mit einer wäßrigen Flüssigkeitsprobe, die möglicherweise den Analyten enthält, derart in Kontakt bringt, daß die Flüssigkeitsprobe von dem ersten über den dritten durch den zweiten Bereich des Trägers hindurchgesaugt wird, und prüft, ob und gegebenenfalls in welchem Ausmaß in dem zweiten Bereich des Trägers ein Signal auftritt.
